# EUROPEAN PATENT SPECIFICATION

(11) **EP 3 243 473 B1**
(45) Date of publication and mention of the grant of the patent: **26.12.2018**
(21) Application number: 17169237.9
(22) Date of filing: 03.05.2017
(51) Int. Cl.: A61B 18/18

(54) **ABLATION INSTRUMENTS WITH A MEMBER HAVING A TRIANGULAR CROSS-SECTION**
ABLATIONSINSTRUMENTE MIT EINEM ELEMENT MIT DREIECKIGEM QUERSCHNITT
INSTRUMENTS D'ABLATION COMPORTANT UN ÉLÉMENT PRÉSENTANT UNE SECTION TRANSVERSALE TRIANGULAIRE

(30) Priority: 05.05.2016 US 201662332080 P; 19.04.2017 US 201715491121
(43) Date of publication of application: 15.11.2017
(73) Proprietor: Covidien LP, Mansfield, MA 02048 (US)
(72) Inventor: Larson, Eric W, Littleton, Colorado 80127 (US); DICKHANS, William, Longmont, Colorado 80503 (US)
(74) Representative: Maschio & Soames IP Ltd

(56) References cited:
- US-A1- 2006 217 693
- US-A1- 2008 033 424
- US-A1- 2012 289 949

## Description

### Background

### 1. Technical Field

The present disclosure relates to ablation instruments and, more specifically, to ablation instruments with a member having a triangular cross-section.

### 2. Discussion of Related Art

Treatment of certain diseases requires the destruction of malignant tissue growths, e.g., tumors. Electromagnetic radiation can be used to heat and destroy tumor cells. Treatment may involve inserting an ablation instrument into tissues where cancerous tumors have been identified. Once the instrument is positioned, electromagnetic energy is passed through the instrument into surrounding tissue.

In the treatment of diseases such as cancer, certain types of tumor cells have been found to denature at elevated temperatures that are slightly lower than temperatures normally injurious to healthy cells. Known treatment methods, such as hyperthermia therapy, heat diseased cells to temperatures above 41° C while maintaining adjacent healthy cells below the temperature at which irreversible cell destruction occurs. These methods involve applying electromagnetic radiation to heat or ablate tissue.

Electrosurgical instruments utilizing electromagnetic radiation have been developed for a variety of uses and applications. Typically, apparatus for use in tissue ablation procedures include a power generation source, e.g., a microwave or radio frequency (RF) electrosurgical generator that functions as an energy source and an ablation instrument for directing energy to the target tissue. The generator and ablation instrument are typically operatively coupled by a cable assembly having a plurality of conductors for transmitting energy from the generator to the instrument and for communicating control signals, feedback and identification signals, between the instrument and the generator.

During tissue ablation procedures, an ablation needle assembly of an ablation instrument is inserted through a trocar to access the surgical site. The trocar is inserted through an opening in tissue to permit access to a surgical site. It is beneficial to decrease the size of the trocar to increase accuracy of the positioning of the trocar and to decrease potential trauma to a patient. The size of the trocar is determined by the instruments (e.g., ablation instrument) that are inserted through the trocar during a given tissue ablation procedure. Reference is made to document US2012/0289949 A1.

Accordingly, there is a continuing need for ablation needle assemblies having a reduced cross-sectional area for insertion through a trocar.

### Summary

This disclosure relates generally to ablation instruments having an ablation needle assembly with a member having a triangular cross-section to reduce the overall cross-sectional area of the ablation needle assembly. The ablation needle assembly includes an outer member and an inner member that cooperate to define inflow and outflow lumens about a feedline. The inflow and outflow lumens circulate a cooling fluid through the needle assembly. One of the inner or outer members has a circular cross-section and the other one of the inner or outer members has a triangular cross-section. The triangular cross-section of one of the inner and outer members may allow for a reduced cross-sectional area of the ablation needle assembly. In addition, the triangular cross-section can provide rigidity to the ablation needle assembly.

In an aspect of the present disclosure, an ablation needle assembly includes an outer member, an inner member, and a feedline. The outer member defines a first lumen that receives the inner member therein. The inner member defines a second lumen that receives the feedline therein. The first and second lumens cooperate to circulate fluid about the feedline. Either the inner or outer member has a triangular cross-section. The other of the inner or outer member may have a non-triangular cross-section. The triangular cross-section of one of the outer or inner member may provide longitudinal stiffness to the ablation needle assembly to prevent longitudinal deflection of the ablation needle assembly.

In aspects, the outer member has a triangular cross-section and the inner member has a circular cross-section. An outer surface of the inner member may be in continuing contact with internal faces of the outer member. The continuing contact of the outer surface of the inner member with the internal faces of the outer member may divide the first lumen into three separate channels. The outer surface of the inner member may have a diameter smaller than a diameter defined by internal faces of the outer member.

In some aspects, the inner member has a triangular cross-section and the outer member has a circular cross section. An inner surface of the outer member may be in continuing contact with external corners of the inner member. The continuing contact of the internal surface of the outer member with the external corners of the inner member separates the first lumen into three separate channels. The feedline may be in continuing contact with internal faces of the inner member. The continuing contact of the feedline with the internal faces of the inner member may separate the second channel into three channels. The feedline may have an outer diameter that is smaller than a diameter defined by the internal faces of the inner member.

In certain aspects, the first and second lumens are configured to circulate a cooling fluid about the feedline. A proximal end of the second lumen may be in fluid communication with an inflow tube and a proximal end of the first lumen may be in fluid communication with an outflow tube. A distal end of the first lumen may be in fluid communication with a distal end of the second lumen.

In another aspect of the present disclosure, an ablation instrument includes a needle assembly, a cable assembly, and a housing. The needle assembly includes an outer member, an inner member, and a feedline. The inner member is disposed within a first lumen that is defined by the outer member. The feedline is disposed within a second lumen that is defined by the inner member. The cable assembly has a connecter at a first end and includes a first conductor. The housing is coupled to a second end of the cable assembly. The housing electrically couples the first conductor to the feed line and fluidly couples an inflow tube with a proximal end of the second lumen and an outflow tube with a proximal end of the first lumen.

In aspects, the cable assembly includes a second conductor that is disposed about the first conductor. The housing may electrically couple the second conductor to the inner member.

Further, to the extent consistent, any of the aspects described herein may be used in conjunction with any or all of the other aspects described herein.

### Brief Description of the Drawings

Various aspects of the present disclosure are described hereinbelow with reference to the drawings, which are incorporated in and constitute a part of this specification, wherein:
FIG. 1 is a schematic diagram of an ablation system including an ablation instrument with a fluid-cooled ablation needle assembly in accordance with an embodiment of the present disclosure;
FIG. 2 is an exploded view, with parts separated, of an ablation needle assembly and a housing of the ablation instrument of FIG. 1;
FIG. 3 is a longitudinal cross-sectional view of the ablation needle assembly and housing of FIG. 2;
FIG. 4 is a cross-sectional view taken along the section line 4-4 of FIG. 3;
FIG. 5 is an enlarged view of the indicated area of detail of FIG. 3;
FIG. 6 is a cross-sectional view of another ablation needle assembly provided in accordance with the present disclosure; and
FIG. 7 is a cross-sectional view of another ablation needle assembly provided in accordance with the present disclosure.

### Detailed Description

The present disclosure is generally directed to ablation systems having an ablation needle assembly with a member having a triangular cross-sectional area to reduce the overall cross-section of the ablation needle assembly. Hereinafter, embodiments of ablation systems with a fluid-cooled ablation needle assembly including the same of the present disclosure are described with reference to the accompanying drawings. Like reference numerals may refer to similar or identical elements throughout the description of the figures. As shown in the drawings and as used in this description, and as is traditional when referring to relative positioning on an object, the term "proximal" refers to that portion of the apparatus, or component thereof, closer to the user and the term "distal" refers to that portion of the apparatus, or component thereof, farther from the user. As it is used in this description, "ablation procedure" generally refers to any ablation procedure, such as, for example, microwave ablation, radiofrequency (RF) ablation, or microwave or RF ablation-assisted resection.

The ablation instrument of the present disclosure may be used in combination with the i-Logic® target identification, navigation, and marker placement systems developed by superDimension™, Ltd and currently marketed by Medtronic. In particular the present disclosure describes devices and systems for the treatment tissue or organs through microwave ablation of targets identified in the patient for treatment. For example, it is contemplated that the systems and methods of the present disclosure may be used to treat liver tissue, kidney tissue, pancreatic tissue, gastrointestinal tissue, interstitial masses, and other portions of the body known to those of skill in the art to be treatable via microwave ablation.

The ablation needle assembly includes an outer member that defines a first lumen, an inner member disposed within the first lumen that defines a second lumen, and a feedline disposed within the second lumen. The first and second lumens are in communication with one another at a distal end of the ablation needle assembly such that cooling fluid circulates through the first and second lumens to cool the feedline. One of the inner or outer members has a circular cross-section and the other has a triangular cross-section to reduce the cross-section of the ablation needle assembly and to provide lateral rigidity to the ablation needle assembly.

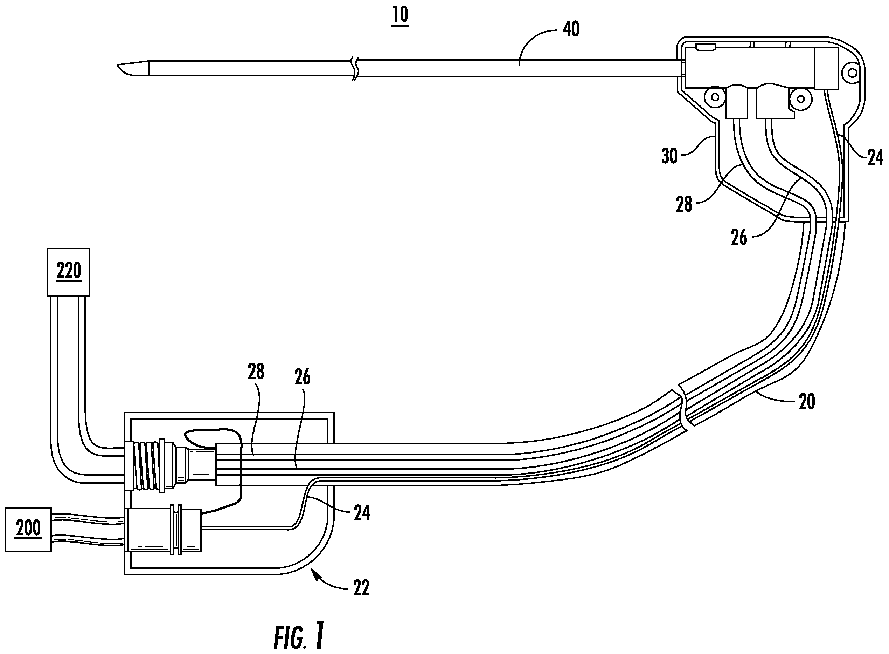

Referring now to FIG. 1, an ablation instrument 10 includes a cable assembly 20, a housing 30, and an ablation needle assembly 40. The cable assembly 20 includes a connector 22, a coaxial cable 24, an inflow tube 26, and an outflow tube 28. The coaxial cable 24 provides electrical communication between the housing 30 and a source of electrosurgical energy 200 which provides the ablation instrument 10 with electrosurgical energy. In addition, the coaxial cable 24 may transmit feedback signals (e.g., reflected power) to the generator 200. The inflow and outflow tubes 26, 28 provide fluid communication between the housing 30 and a source of cooling fluid 220.

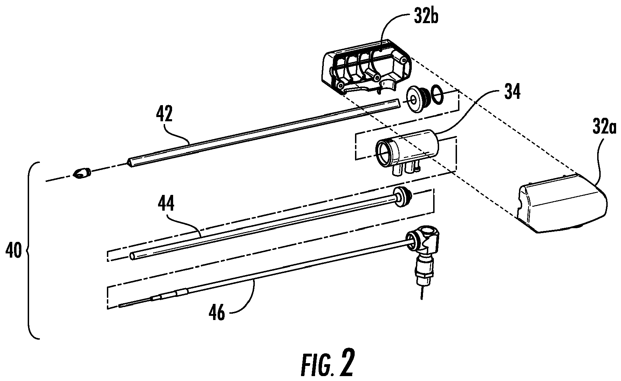

Referring also to FIG. 2, the housing 30 includes two half-shells 32a, 32b which couple together around a hub 34 that interconnects the inflow tube 26 and the outflow tube 28 with the ablation needle assembly 40.

With reference to FIGS. 1-3, an embodiment of an ablation needle assembly 40 is described in accordance with the present disclosure. The ablation needle assembly 40 includes an outer member 42, an inner member 44, and a feedline 46. The feedline 46 may terminate in a radiating section including a balun, a proximal radiating portion, a distal radiating portion, and a feed gap. These components and their interrelation define the active heating zone of ablation instrument 10. Exemplary feedlines and their interrelated components are described in U.S. Patent Publication No. 2014/0290830, entitled "Step-Down Coaxial Microwave Ablation Applicators and Methods for Manufacturing Same," filed March 28, 2014 by Joseph D. Brannan, and U.S. Patent No. 9,301,723, entitled "Microwave Energy Device and System," filed March 15, 2013 by Brannan et al. the entire contents of which are hereby incorporated by reference.

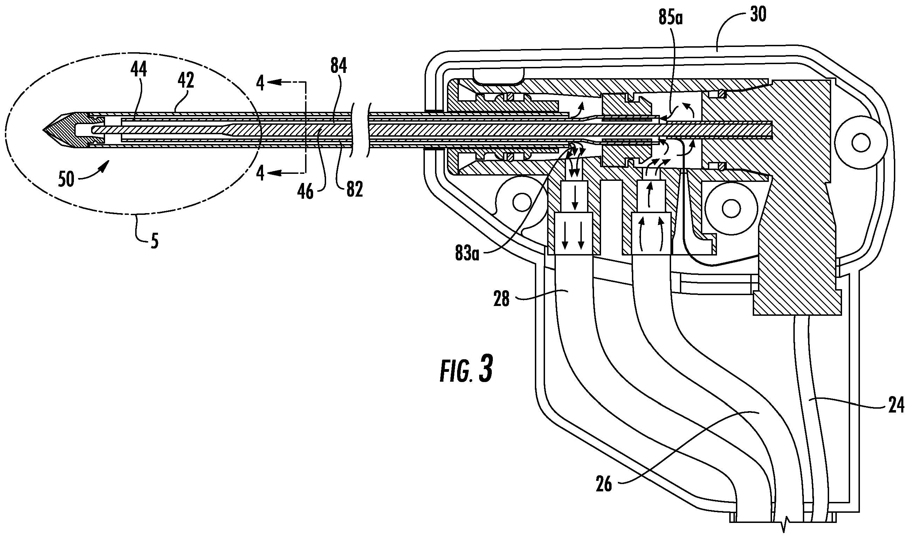

With particular reference to FIG. 3, within the housing 30, a proximal end 83a, a first lumen 82 is in fluid communication with the inflow tube 26 or the outflow tube 28 and a proximal end 85a of a second lumen 84 is in fluid communication with the other one of the inflow tube 26 or the outflow tube 28. As shown, the first lumen 82 is in fluid communication with the outflow tube 28 such that the first lumen 82 is an outflow lumen and the second lumen 84 is in fluid communication with the inflow tube 26 such that the second lumen is an inflow lumen.

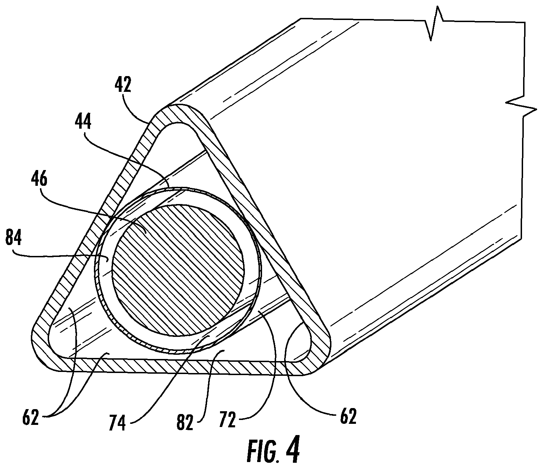

Referring also to FIG. 4, the outer member 42 is triangular in cross-section and includes three internal faces 62 which together define the first lumen 82. The inner member 44 is disposed within the first lumen 82 and is circular in cross-section. As shown, the inner member 44 is sized such that an outer surface 72 of the inner member 44 contacts each of the internal faces 62 of the outer member 42. The contact between the inner member 44 and the outer member 42 may divide the first lumen 82 into three separate channels. Alternatively, the inner member 44 may be sized such that the inner member 44 floats within the first lumen 82 such that the outer surface 72 is in intermittent contact with one or more of the internal faces 62 of the outer member 42. It is envisioned that floating the inner member 44 within the first lumen 82 may reduce or eliminate forces or stresses resulting in placement of the ablation needle assembly 40 within a patient from being transmitted to the inner member 44 and/or the feedline 46. Reducing or eliminating forces or stresses may reduce breaking of the inner member 44 and/or the feedline 46.

The inner member 44 includes an inner surface 74 that defines the second lumen 84. The feedline 46 is disposed within the second lumen 84 of the inner member 44. As shown, the feedline 46 floats within the second lumen 84 and may intermittently contact portions of the inner surface 74 of the inner member 44.

It will be appreciated that the triangular cross-section of the outer member 42 reduces the overall size (i.e., diameter) of the cross-section of the outer member 42 when compared to an outer member having a circular cross-section. In addition, the triangular cross-section of the outer member 42 provides lateral stiffness to the ablation needle assembly 40. It is envisioned that the cross-sectional area of the inner and/or outer lumens 82, 84 may be larger when one of the inner or outer members 42, 44 has a triangular cross-section allowing greater fluid flow. The greater fluid flow may result in increased cooling of the feedline 46 such that the ablation needle assembly 40 may deliver a greater amount energy to tissue when compared to an ablation needle assembly having inner and outer members having a similar cross-section to one another and a similar overall diameter to the ablation needle assembly 40.

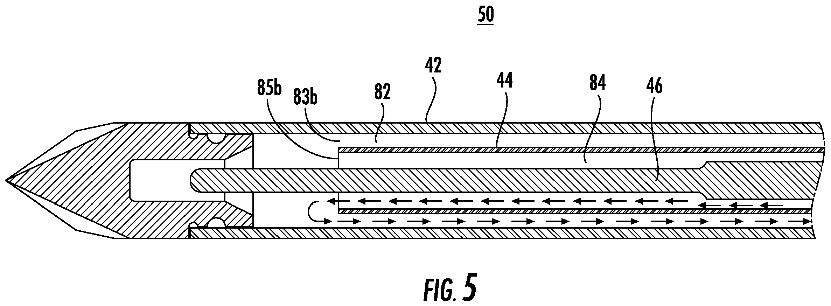

Referring to FIG. 5, within the ablation needle assembly 40, a distal end 83b of the first lumen 82 is in fluid communication with a distal end 85b of the second lumen 84. As such, the inflow tube 26 is in fluid communication with the outflow tube 28 via the first and second lumens 82, 84 such that fluid entering the ablation needle assembly 40 through the inflow tube 26 exits through the outflow tube 28. As the fluid circulates through the ablation needle assembly 40, the fluid may absorb heat from the inner member 44 and/or the feedline 46.

It is envisioned that circulating fluid through the ablation needle assembly 40 may create a uniform dielectric constant about a radiating portion of the feedline 46 which can generate spherical ablations and can improve the efficiency of energy transfer from microwave to thermal, particularly in the near field. Examples of systems and methods for generating spherical ablations are disclosed in U.S. Patent Application No. 14/831,467, entitled "Systems and Methods for Spherical Ablations," filed August 20, 2015 by Joseph D. Brannan, the entire contents of which are hereby incorporated by reference.

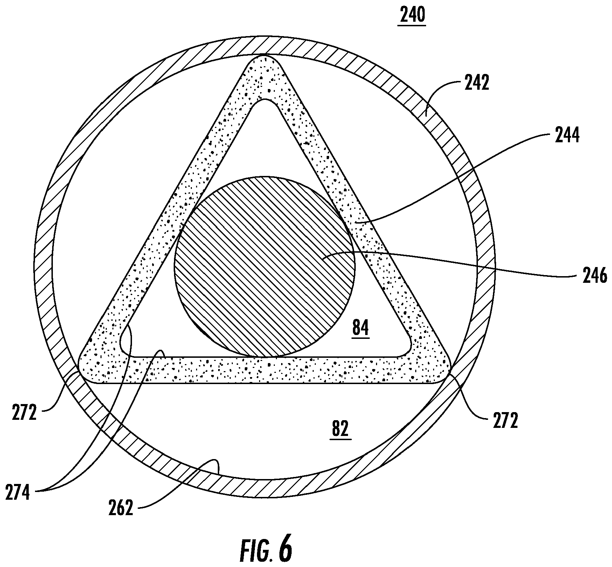

With reference to FIG. 6, another embodiment of an ablation needle assembly 240 is described in accordance with the present disclosure and includes an outer member 242, an inner member 244, and a feedline 246. The outer member 242 is circular in cross-section and includes an internal surface 262 which defines the first lumen 82. The inner member 244 is disposed within the first lumen 82. The inner member 244 is triangular in cross-section with outer corners 272 and is sized such that the outer corners 272 each contact the internal surface 262 of the outer member 242. The contact between the outer corners 272 of the inner member 244 with the internal surface 262 of the outer member 242 may divide the first lumen 82 into three separate channels. It is contemplated that the inner member 244 may be sized such that the inner member 244 floats within the first lumen 82 with the outer corners 272 in intermittent contact with the internal surface 262 of the outer member 242. The inner member 244 includes three internal faces 274 that together define the second lumen 84. The feedline 246 is disposed within the second lumen 84 of the inner member 244. As shown, the feedline 246 is disposed within the second lumen 84 with the feedline 246 in contact with each of the internal faces 274 of the inner member 244. The contact of the feedline 246 with the internal faces 274 of the inner member 244 may divide the second lumen 84 into three separate channels. Alternatively, the feedline 246 may float within the second lumen 84 and may intermittently contact portions one or more of the internal faces 274 of the inner member 244.

The triangular cross-section of the inner member 244 of the needle assembly 240 reduces the overall cross-sectional area of the outer member 242 required to carry a feedline when compared to a needle assembly having inner and outer members each having a circular cross-section. Specifically, the diameter of the outer member 242 can be reduced while maintaining fluid lumens (e.g., first and second lumens 82, 84) between the feedline 246 and the inner member 244 and between the inner member 244 and the outer member 242. In addition, the triangular cross-section of the inner member 244 may provide longitudinal stiffness to the needle assembly 240.

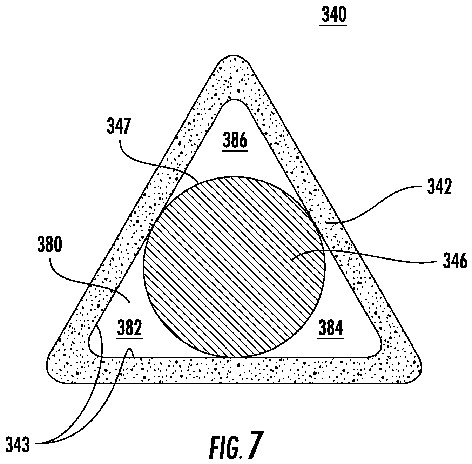

Referring to FIG. 7, another embodiment of an ablation needle assembly 340 is described in accordance with the present disclosure and includes an outer member 342 and a feedline 346. The outer member 342 has a triangular cross-section and defines a lumen 380. The feedline 346 has a circular cross-section and is disposed within the lumen 380 defined by the outer member 342. An outer surface 347 of the feedline 346 is in continuing contact with inner faces 343 of the outer member 342 such that the lumen 380 defined by the outer member is divided into three separate channels 382, 384, 386. A proximal end of one of the channels (e.g., channel 382) may be in fluid communication with an inflow tube such that the channel is an inflow channel. A proximal end of another one of the channels (e.g., channel 384) may be in fluid communication with an outflow tube such that the channel is an outflow channel. Distal ends of the inflow and outflow channels are in fluid communication with one another such that fluid can flow from the inflow tube to the outflow tube about the feedline 342.

Although embodiments have been described in detail with reference to the accompanying drawings for the purpose of illustration and description, it is to be understood that the inventive processes and apparatus are not to be construed as limited thereby. It will be apparent to those of ordinary skill in the art that various modifications to the foregoing embodiments may be made without departing from the scope of the disclosure.

## Claims

1. An ablation needle assembly (40), comprising:
an outer member (42) defining a first lumen (82);
an inner member (44) disposed within the first lumen and defining a second lumen;
and
a feedline (46) disposed within the second lumen, wherein the first lumen and the second lumen cooperate to circulate fluid about the feedline,
wherein the outer member has a triangular cross-section and the inner member has a circular cross-section, and the inner member has an outer surface (72) in continuing contact with internal faces of the outer member (42).

2. The ablation needle assembly according to claim 1, wherein the continuing contact of the outer surface (72) of the inner member (44) with the internal faces of the outer member (42) divides the first lumen into three separate channels.

3. An ablation needle assembly (240), comprising:
an outer member (242) defining a first lumen (82);
an inner member (244) disposed within the first lumen and defining a second lumen (84); and
a feedline (246) disposed within the second lumen, wherein the first lumen and the second lumen cooperate to circulate fluid about the feedline,
wherein the inner member has a triangular cross-section and the outer member has a circular cross-section.

4. The ablation needle assembly according to claim 3, wherein the outer member (244) has an internal surface (262), the internal surface in continuing contact with external corners (272) of the inner member.

5. The ablation needle assembly according to claim 4, wherein the continuing contact of the internal surface (262) of outer member (242) with the external corners (272) of the inner member (244) separates the first lumen (82) into three channels.

6. The ablation needle assembly according to claim 3, 4 or 5, wherein the feedline (246) is in continuing contact with internal faces (274) of the inner member (244).

7. The ablation needle assembly according to claim 6, wherein the continuing contact of the feedline (246) with the internal faces (274) of the inner member (244) separates the second lumen (84) into three channels.

8. The ablation needle assembly according to claim 3, 4 or 5, wherein the feedline (246) has an outer diameter smaller than a diameter defined by the internal face(s) (274) of the inner member (244).

9. The ablation needle assembly according to any preceding claim, wherein the first (82) and second (84) lumens are configured to circulate a cooling fluid about the feedline.

10. The ablation needle assembly according to claim 9, wherein a proximal end of the second lumen (84) is in fluid communication with an inflow tube (26) and a proximal end of the first lumen (82) is in fluid communication with an outflow tube (28).

11. The ablation needle assembly according to claim 9, wherein a distal end of the first lumen (82) is in fluid communication with a distal end of the second lumen (84).

12. The ablation needle assembly according to claim 1 or 2, wherein the triangular cross-section of the outer member provides longitudinal stiffness to the ablation needle assembly to prevent longitudinal deflection of the ablation needle assembly.

## Patentansprüche

1. Ablationsnadelanordnung (40), umfassend:
ein äußeres Element (42), das ein erstes Lumen (82) definiert;
ein inneres Element (44), das in dem ersten Lumen angeordnet ist und ein zweites Lumen definiert; und
eine Zuleitung (46), die innerhalb des zweiten Lumens angeordnet ist, wobei das erste Lumen und das zweite Lumen zusammenwirken, um Fluid um die Zuleitung herum zu zirkulieren,
wobei das äußere Element einen dreieckigen Querschnitt aufweist und das innere Element einen kreisförmigen Querschnitt aufweist und wobei das innere Element eine äußere Oberfläche (72) aufweist, die in fortlaufendem Kontakt mit Innenseiten des äußeren Elements (42) steht.

2. Ablationsnadelanordnung nach Anspruch 1, wobei der fortlaufende Kontakt der äußeren Oberfläche (72) des inneren Elements (44) mit den Innenseiten des äußeren Elements (42) das erste Lumen in drei separate Kanäle unterteilt.

3. Ablationsnadelanordnung (240), umfassend:
ein äußeres Element (242), das ein erstes Lumen (82) definiert;
ein inneres Element (244), das in dem ersten Lumen angeordnet ist und ein zweites Lumen (84) definiert; und
eine Zuleitung (246), die innerhalb des zweiten Lumens angeordnet ist, wobei das erste Lumen und das zweite Lumen zusammenwirken, um Fluid um die Zuleitung herum zu zirkulieren,
wobei das innere Element einen dreieckigen Querschnitt aufweist und das äußere Element einen kreisförmigen Querschnitt aufweist.

4. Ablationsnadelanordnung nach Anspruch 3, wobei das äußere Element (244) eine innere Oberfläche (262) aufweist, wobei die innere Oberfläche in fortlaufendem Kontakt mit äußeren Ecken (272) des inneren Elements steht.

5. Ablationsnadelanordnung nach Anspruch 4, wobei der fortlaufende Kontakt der innere Oberfläche (262) des äußeren Elements (242) mit den äußeren Ecken (272) des inneren Elements (244) das erste Lumen (82) in drei separate Kanäle unterteilt.

6. Ablationsnadelanordnung nach Anspruch 3, 4 oder 5, wobei die Zuleitung (246) in fortlaufendem Kontakt mit Innenseiten (274) des inneren Elements (244) steht.

7. Ablationsnadelanordnung nach Anspruch 6, wobei der fortlaufende Kontakt der Zuleitung (246) mit den Innenseiten (274) des inneren Elements (244) das zweite Lumen (84) in drei Kanäle unterteilt.

8. Ablationsnadelanordnung nach Anspruch 3, 4 oder 5, wobei die Zuleitung (246) einen äußeren Durchmesser aufweist, der kleiner als ein Durchmesser ist, der durch die Innenseite(n) (274) des inneren Elements (244) definiert wird.

9. Ablationsnadelanordnung nach einem vorstehenden Anspruch, wobei das erste (82) und das zweite (84) Lumen konfiguriert sind, um ein Kühlfluid um die Zuleitung herum zu zirkulieren.

10. Ablationsnadelanordnung nach Anspruch 9, wobei ein proximales Ende des zweiten Lumen (84) mit einem Einströmrohr (26) in Fluidverbindung steht und ein proximales Ende des ersten Lumen (82) mit einem Ausströmrohr (28) in Fluidverbindung steht.

11. Ablationsnadelanordnung nach Anspruch 9, wobei ein distales Ende des ersten Lumen (82) mit einem distalen Ende des zweiten Lumen (84) in Fluidverbindung steht.

12. Ablationsnadelanordnung nach Anspruch 1 oder 2, wobei der dreieckige Querschnitt des äußeren Elements der Ablationsnadelanordnung Längssteifigkeit verleiht, um eine Längsauslenkung der Ablationsnadelanordnung zu verhindern.

## Revendications

1. Ensemble formant aiguille d'ablation (40), comprenant :
un élément extérieur (42) définissant une première lumière (82) ;
un élément intérieur (44) disposé à l'intérieur de la première lumière et définissant une seconde lumière ; et
une ligne d'alimentation (46) disposée à l'intérieur de la seconde lumière, dans lequel la première lumière et la seconde lumière coopèrent pour faire circuler un fluide autour de la ligne d'alimentation,
dans lequel l'élément extérieur a une section transversale triangulaire et l'élément intérieur a une section transversale circulaire, et l'élément intérieur a une surface extérieure (72) en contact ininterrompu avec des faces internes de l'élément extérieur (42).

2. Ensemble formant aiguille d'ablation selon la revendication 1, dans lequel le contact ininterrompu de la surface extérieure (72) de l'élément intérieur (44) avec les faces internes de l'élément extérieur (42) divise la première lumière en trois canaux distincts.

3. Ensemble formant aiguille d'ablation (240), comprenant :
un élément extérieur (242) définissant une première lumière (82) ;
un élément intérieur (244) disposé à l'intérieur de la première lumière et définissant une seconde lumière (84) ; et
une ligne d'alimentation (246) disposée à l'intérieur de la seconde lumière, dans lequel la première lumière et la seconde lumière coopèrent pour faire circuler un fluide autour de la ligne d'alimentation,
dans lequel l'élément intérieur a une section transversale triangulaire et l'élément extérieur a une section transversale circulaire.

4. Ensemble formant aiguille d'ablation selon la revendication 3, dans lequel l'élément extérieur (244) a une surface interne (262), la surface interne étant en contact ininterrompu avec des angles externes (272) de l'élément intérieur.

5. Ensemble formant aiguille d'ablation selon la revendication 4, dans lequel le contact ininterrompu de la surface interne (262) d'élément extérieur (242) avec les angles externes (272) de l'élément intérieur (244) sépare la première lumière (82) en trois canaux.

6. Ensemble formant aiguille d'ablation selon la revendication 3, 4 ou 5, dans lequel la ligne d'alimentation (246) est en contact ininterrompu avec des faces internes (274) de l'élément intérieur (244).

7. Ensemble formant aiguille d'ablation selon la revendication 6, dans lequel le contact ininterrompu de la ligne d'alimentation (246) avec les faces internes (274) de l'élément intérieur (244) sépare la seconde lumière (84) en trois canaux.

8. Ensemble formant aiguille d'ablation selon la revendication 3, 4 ou 5, dans lequel la ligne d'alimentation (246) a un diamètre extérieur plus petit qu'un diamètre défini par la ou les face(s) interne(s) (274) de l'élément intérieur (244).

9. Ensemble formant aiguille d'ablation selon n'importe quelle revendication précédente, dans lequel la première (82) et la seconde (84) lumières sont configurées pour faire circuler un fluide de refroidissement autour de la ligne d'alimentation.

10. Ensemble formant aiguille d'ablation selon la revendication 9, dans lequel une extrémité proximale de la seconde lumière (84) est en communication à fluide avec un tube d'afflux (26) et une extrémité proximale de la première lumière (82) est en communication à fluide avec un tube d'écoulement (28).

11. Ensemble formant aiguille d'ablation selon la revendication 9, dans lequel une extrémité distale de la première lumière (82) est en communication à fluide avec une extrémité distale de la seconde lumière (84).

12. Ensemble formant aiguille d'ablation selon la revendication 1 ou 2, dans lequel la section transversale triangulaire de l'élément extérieur procure une rigidité longitudinale à l'ensemble formant aiguille d'ablation pour empêcher un débattement longitudinal de l'ensemble formant aiguille d'ablation.
